# EUROPEAN PATENT SPECIFICATION

(11) **EP 0 914 082 B1**
(45) Date of publication and mention of the grant of the patent: **31.10.2001**
(21) Application number: 97930424.3
(22) Date of filing: 23.06.1997
(51) Int. Cl.: A61K 7/32, A61K 7/48

(54) **ANTIPERSPIRANT/DEODORANT FLUID COMPOSITION**
ANTITRANSPIRANT/DEODORANT FLÜSSIGE ZUSAMMENSETZUNG
COMPOSITIONS FLUIDES ANTISUDORALES ET DEODORANTES

(30) Priority: 28.06.1996 US 20743 P; 30.10.1996 GB 9622582; 30.10.1996 GB 9622581
(43) Date of publication of application: 12.05.1999
(73) Proprietor: UNILEVER PLC, London EC4P 4BQ (GB); UNILEVER N.V., 3013 AL Rotterdam (NL)
(72) Inventor: BREWSTER, David, Allen, Shelton, CT 06484 (US); OROFINO, Steven, Anthony, Stratford, CT 06497 (US); DOBKOWSKI, Brian, John, Shelton, CT 06484 (US); JONES, Francis, Guilford, CT 06437 (US); EDWARDS, Christopher John Carruthers, Leeds LS22 5BU (GB); ESSER, Isabelle Claire Helene Marie, Wirral, Merseyside L62 5EB (GB)
(74) Representative: Pearce, Timothy
(86) International application number: EP9703370
(87) International publication number: WO9800097

(56) References cited:
- EP-A- 0 295 886
- EP-A- 0 473 039
- US-A- 5 654 362

## Description

The invention relates to underarm compositions having deodorancy and/or perspiration inhibiting properties.

Deodorant/antiperspirant compositions in the form of creams are known in the art. Primarily they have been oil-in-water or water-in-oil emulsions. Although these provide convenient vehicles for delivery of deodorant/antiperspirant actives, emulsions generally tend to produce undesirable sensations on the skin. They are sticky and feel wet. Oil continuous emulsions require shaking and feel oily upon application. Any water system also requires time to dry on the skin.

According to a further aspect, the invention relates to liquid antiperspirant compositions containing a siloxane suspending agent especially useful for roll-on, pump and aerosol antiperspirant compositions.

Anhydrous antiperspirant/deodorant creams have been reported in U.S. Patent 5,102,656 (Kasat). A creamy, heterogeneous anhydrous product is described whose ingredients include a volatile silicone carrier, a gelling agent such as castorwax and a physiologically acceptable antiperspirant agent.

Cream type products are also described in U.S. Patent 5,069,897 (Orr) and U.S. Patent 4,840,789 (Orr et al.).

Both of these patents formulate a cream with a stickiness or greasiness, rapid drying time, better spreadability and effectiveness as a deodorant active.

Further, it is known for anhydrous underarm compositions for topical application to contain non-volatile silicone fluids such as polyorganosiloxanes which impart emolliency to the composition and can provide a masking effect to conceal solids present in the composition. Examples of such solids include antiperspirant actives. The efficacy of the composition is not seriously affected by the presence of the non-volatile silicone. An example of such compositions is to be found in EP 28853 (The Procter & Gamble Company).

Many such compositions also contain volatile silicones as carrier fluids. However, volatile silicones tend to exhibit syneresis and leak from the packaged underarm composition. Such leakage is undesirable and perceived as a serious consumer negative.

It is also known to include certain categories of alkyl siloxane waxes in underarm compositions. EP 549223 (Dow Corning) describes stick, roll on and spray underarm compositions containing certain long chain alkyl silicone waxes. The waxes are alleged to provide the formulations with desirable characteristics such as improved hardness, reduced whitening, improved skin feel and compatibility with other ingredients.

Our co-pending British Patent Application Number 9506039.8 describes the use of alkyl ester siloxane waxes to improve the sensory properties such as skin feel of underarm compositions.

In particular in the field of liquid antiperspirant compositions, anhydrous suspensions of antiperspirant active are widely used in the art. However, many suspension agents while suspending the antiperspirant active in a delivery vehicle can cause staining and also undesirable sensory attributes in the composition e.g. wetness while separation can still result. A disadvantage associated with all anhydrous suspension liquid antiperspirant formulations is the tendency of settling to take place despite the presence of a bentonite or silicate suspending/thickening agent.

Moreover, silicate suspending agents when used at high levels in antiperspirant formulations can thicken the carrier and masking liquids excessively and disrupt processing methods. However, when the levels of silica used are too low separation over time can occur thereby requiring shaking of the formulation prior to use.

An object of the invention is to provide an underarm composition which overcomes the disadvantages of the formulations of the prior art.

Still another object of the present invention is to provide an underarm cream exhibiting a reduced level of syneresis and thereby avoiding any leakage of product from its packaging.

These and other objects of the present invention will become more readily apparent from review of the subsequent summary, detailed description and examples.

According to the first aspect of the invention, there is provided a fluid underarm treatment composition is provided comprising:
(I) an underarm active present in an effective amount to inhibit odour or to reduce perspiration;
(ii) from 0.1 to 30% by weight of a cross-linked non-emulsifying siloxane elastomer;
(iii)from 10 to 80% by weight of a volatile siloxane.

According to further aspect of the invention, there is provided a cream underarm treatment composition comprising:
(I) an deodorant/antiperspirant active present in an effective amount to inhibit odour or to reduce perspiration;
(ii) from 0.1 to 50% by weight of a cross-linked or partially cross-linked non-emulsifying siloxane elastomer;
(iii)from 10 to 80% by weight of a volatile siloxane.

According to yet a further aspect of the invention, there is provided an anhydrous fluid suspension antiperspirant composition suitable for topical application to human skin, comprising:
I. an effective amount of an antiperspirant astringent;
ii. a volatile silicone; and
iii. a cross-linked or partially cross-linked non-emulsifying siloxane elastomer.

Where the composition is a suspension, more specifically the invention provides a fluid suspension antiperspirant composition suitable for topical application to the human skin, comprising:
I. 5-25% by weight of the total composition of an antiperspirant astringent;
ii. 20-90% by weight of the total composition of a linear or cyclic volatile silicone;
iii. 0.1 to 20% by weight of a dimethicone/vinyldimethicone cross polymer elastomer thickening/suspending agent, and
iv. a masking oil.

In yet a further embodiment of the invention, there is provided an aerosol antiperspirant composition characterised in that it comprises from 7 to 60% of a suspension composition described above, and from 40 to 93% propellant.

Now it has been discovered that an ultradry deodorant/antiperspirant cream of enhanced properties can be achieved through incorporation of a cross-linked or partially cross linked non-emulsifying siloxane elastomer in combination with a volatile silicone. One of the main advantages is that when the elastomer swells in the volatile siloxane (e.g. cyclomethicone), the resultant composition does not cause syneresis that would normally cause leakage problems. Furthermore, the elastomer thickens the cyclomethicone thus eliminating the need for other thickeners that could reduce efficacy. Generally the aesthetics of the resultant cream product are superior to those of commercial products. Application is also smooth and dry.

Preferably the cross-linked siloxane elastomer is formed from the hydrosilation of vinyl silicone fluids by hydrosiloxane or MQ hydride fluids.

When the fluid composition is in the form of a cream, suitably the cream has a cone penetration value ranging from 2 mm to 36 mm. The underarm compositions of the present invention which are creams may typically have a cone penetration value ranging from 2 from 36 mm, preferably 10 to 36 mm, more preferably from 10 to 25 mm, optimally from 12 to 20 mm as measured in the Standard Test Method for Cone Preparation of petrolatum (ASTM D 937).

A first essential element of compositions according to the present invention is that of a deodorant and/or antiperspirant active. Most preferable is an astringent salt which combines the properties of deodorancy and antiperspirancy. Suitable astringents may be inorganic or organic salts of aluminum, zirconium, zinc and mixtures thereof. Salts useful as astringents or a components of astringent complexes include aluminum halides, aluminum hydroxyhalides, zirconyl oxyhalides, zirconyl hydroxyhalides and mixtures of these salt materials.

In particular, typically used antiperspirant salts include inorganic and organic salts of aluminium and zirconium and mixtures thereof. Particularly preferred are the aluminium/zirconium salts of aluminium halides, aluminium hydroxyhalides, aluminium zirconium salts and mixtures thereof. Particularly preferred antiperspirant salts include activated aluminium chlorohydrate compounds as described in EP6,739 (Unilever NV et al). Further antiperspirant actives are described in EP 28,853. The contents of both these applications are incorporated herein by reference.

Aluminum salts of this type include aluminum chloride and the aluminum hydroxyhalides having the general formula Al₂(OH)ₓQ_{y}-XH₂O where Q is chlorine, bromine or iodine, where x is 2 to 5 and x+y=6 and x and y do not need to be integers; and where x is about 1 to 6.

Zirconium compounds which are useful may be represented by the following general empirical formula: ZnO(OH)_{2-nz}B_{z} wherein z may vary from about 0.9 to 2 and need not be an integer, n is the valence of B, 2-nz is greater than or equal to 0, and B may be selected from the group consisting of halides, nitrate, sulfamate, sulfate and mixtures thereof. As with the basic aluminum compounds, it will be understood that the aforementioned formula is greatly simplified and is intended to represent and include compounds having coordinated and/or bound water in various quantities, as well as polymers, mixtures and complexes of the above. Zirconium hydroxy salts actually represent a range of compounds having various amounts of the hydroxy group, varying from about 1.1 to only slightly greater than 0 groups per molecule.

Several types of antiperspirant complexes utilizing the above astringent salts are known in the art. For example, U.S. Patent 3,792,068 (Luedders et al.), discloses complexes of aluminum, zirconium and amino acids such as glycerine. Complexes reported therein and similar structures are commonly known as ZAG. The ZAG complexes ordinarily have an Al:Zr ratio of from about 1.67 to 12.5 and a metal:Cl ratio of from about 0.73 to 1.93. A preferred aluminum compound for preparation of ZAG type complexes is aluminum chlorhydroxide of the empirical formula Al₂(OH)₅Cl·2H₂O. Preferred zirconium compounds for preparation of ZAG-type complexes are zirconyl hydroxychloride having the empirical formula ZrO(OH)Cl·3H₂O and the zirconyl hydroxyhalides of the empirical formula ZrO(OH)₂₋ₐCl₂·nH₂O wherein a is from 1.5 to 1.87 and n is from about 1 to 7. The preferred amino acid for preparing such ZAG-type complexes is glycine of the formula CH₂(NH₂)COOH. Spherical ZAG, with particle size 1 to 100 microns, is especially preferred.

Amounts of the deodorant active may range from 0.1 to 70%. When the active is an astringent salt, the amounts may range from about 15% to 60% by weight calculated on an anhydrous metal salt basis (exclusive of glycine, the salts of glycine or other complexing agents).

Deodorant actives according to the present invention also include materials other than those functioning as antiperspirants. Deodorants should be capable of killing or hindering the growth of microorganisms that generate malodour or that promote the decomposition of body oils into odiferous fatty acids. Most prominent among organic antimicrobial materials are short chain monohydric alcohols, polyhydric alcohols, triclosan, triclorban, chlorhexedine and certain fragrant oils known as deo perfumes (e.g. U.S. Patent 4,278,658 to Hooper et al.). Amounts of the organic antimicrobial materials may range from 0.1 to 1%, preferably 0.2 to 0.5% by weight. Inorganic antimicrobial materials may also serve as deodorant actives. These include zinc oxide, zinc hydroxide, zinc carbonate, zinc phenolsulfonate, zinc ricinoleate, magnesium oxide, magnesium hydroxide, magnesium carbonate, sodium bicarbonate, lanthanum oxide, lanthanum hydroxide, lanthanum carbonate and combinations thereof.

Crosslinked non-emulsifying siloxane elastomers are the second essential elements of this invention. They will have an average number molecular weight in excess of 10,000, preferably in excess of 1,000,000 and optimally will range from 10,000 to 20 million. The term "non-emulsifying" defines a siloxane from which polyoxyalkylene units are absent. Illustrative of the elastomer is a material with the CTFA name of Crosslinked Stearyl Methyl-Dimethyl Siloxane Copolymer, available as Gransil SR-CYC (25-35% active elastomer) from Grant Industries, Inc., Elmwood Park, New Jersey. Supply of related elastomer may also be available from the General Electric Company.

The silicone elastomers are crosslinked or partially crosslinked, entangled, viscoelastic polymer networks made by the Pt catalysed reaction known as hydrosilation of vinyl silicone fluids by either hydrosiloxane fluids or highly branched MQ hydride fluids. Control of the stoichiometry and type of the vinyl silicone fluid and the silanic crosslinker controls the properties of the cured networks. Additional vinyl reactants such as vinylalkenes can be introduced in the reactive medium to further modify the silicone network. The choice of the reaction solvent(s) is also a means to modify the properties of the resultant gels as a certain amount, which can easily be controlled, will be entrapped into the polymeric network giving different properties such as skin feel. The average molecular weight of the silicone elastomers is between 10,000 and 20 million.

Amounts of the elastomer may range from 0.1 to 30%, optimally from 1 to 15%, most preferably from 3 to 10% by weight.

A third essential element to be incorporated into the compositions of this invention is that of a volatile siloxane. This material may be present in amounts from 10 to 80%, preferably from 10 to 60%, optimally from 30 to 50% by weight.

The term "volatile" refers to those materials having a measurable pressure at ambient conditions. Volatile polyorganosiloxanes useful herein may be cyclic or linear. Preferred cyclic silicones include polydimethylsiloxanes containing from about 3 to about 9 silicon atoms, preferably about 3-7 silicone atoms, more preferably from about 4 to about 5 silicon atoms, generally known as cyclomethicones. Preferred linear silicone oils include the polydimethylsiloxanes containing from about 3 to about 9 silicon atoms. The linear volatile silicones generally have viscosities of less than about 5 centistokes at 25°C, while the cyclic materials have viscosities of less than about 10 centistokes, the preferable range being from 0.1 to 8 centistokes. Examples of silicone oils useful in the present invention include: Dow Corning 344, Dow Corning 345 and Dow Corning 200 (manufactured by the Dow Corning Corporation); Silicone 7207 and Silicone 7158 (manufactured by the Union Carbide Corporation); SF1202 (manufactured by General Electric).

A principal advantage of the invention is that when the elastomer is swollen in the volatile siloxane (e.g. cyclomethicone), and the resultant material ( hereinafter referred to as the "gel") prevents syneresis that would normally give rise to leakage problems. Furthermore, the elastomer thickens the cyclomethicone thus eliminating or partially eliminating the need for other thickeners that could reduce efficacy. The aesthetics of the resultant cream product are superior to those of known products. Application is also smooth and dry.

Typically the crosslinked silicone polymeric networks are swollen substantially by oils preferably silicone fluids such as cyclomethicone and/or dimethicone to form gels, the characteristics of the gel being dependant on the degree of crosslinking. The resultant gels are not weakened by normal shearing (e.g at 2000rpm), heat or rubbing on the skin and contain between 0.1 and 50% of crosslinked silicone polymeric network i.e. elastomer.

Illustrative examples of gels are materials with the CTFA name of cyclomethicone dimethicone/vinyl dimethicone crosspolymer containing about 0.1 to 50%, preferably 1 to 20% and more preferably 1% to 8% of the dimethicone/vinyl dimethicone crosspolymer ( elastomer) and known as KSG-15 ex Shin-Etsu. Other such suitable crosslinked silicone elastomers and gels are available from Witco Corporation, Dow Corning and General Electric.

Compositions of the present invention may also contain a powdered filler. Illustrative of this category are starches, talc, fumed silica (e.g. Cab-O-Sil from the Cabot Corporation), finely divided silica (e.g. sodium silicate), sodium bicarbonate, magnesium aluminium silicate, clays and mixtures thereof. Most preferred and effective are corn starch and modified starches, especially aluminum starch octenyl succinate, commercially available from the National Starch & Chemical Company under the trademark Dry Flo® .

Amounts of the powdered filler will range from 1 to 40%, preferably from 10 to 35%, optimally from 15 to 30% by weight.

Inert particulates may also be included in cosmetic creams of the present invention. Illustrative of such materials are the polyolefins (such as polyethylene and polypropylene) and nylon. Most preferred are the spherical or non-spherical polyethylene powders. Amounts of these materials may range from 0.1 to 20%, preferably from 1 to 10% by weight.

Optionally included within the cosmetic creams of the present invention is that of a non-volatile C₁₂-C₄₀ hydrocarbon. Amounts of this material may range from 1 to 40%, preferably from 5 to 25%, optimally from 10 to 20% by weight.

The non-volatile hydrocarbon should have a viscosity of at least 10 cs at 25°C, preferably ranging from 10 to 100,000 cs at 25°C. The C₁₂-C₄₀, preferably C₂₀-C₄₀ hydrocarbon may either be saturated or unsaturated. Examples include dodecane, tridecane, tetradecane, pentadecane, hexadecane, heptadecane, octadecane, nonadecane, elcosane, heneicosane, docosane, tricosane, tetracosane, pentacosane, isomers of these compounds and mixtures thereof. Most preferred is polydecene available from the Ethyl Corporation under the Ethylflo trademark.

The polyolefins are suitably hydrocarbon polymers, with the preferred ones being liquid at room temperature (i.e. 21°C). It is also highly preferred that the poly olefin in the composition has a relatively low viscosity. Preferably, the viscosity of the poly olefin hydrocarbon masking agent is less than about 40 cSt at 40°C, more preferably less than about 30 cSt at 40°C.

Preferably, the poly olefin comprises a poly alpha olefin. Preferred poly alpha olefins for use in compositions according to the invention are polydecenes, for example the Silkflo range of polydecenes, manufactured by Albermarle Corporation. Other preferred poly olefins for use in compositions according to the invention include polybutene, which is commercially available under the trade name Panalene L14E from Amoco, and polyisobutene, which can be obtained from Prespere under the trade name Permethyl.

As such, preferred poly olefins for use in compositions according to the invention may have monomer chain lengths in the region of 3-15 carbon atoms. Preferred poly olefin blends which are commercially available may conveniently contain a blend of various polymers, including dimers, trimers, and so on. Preferred materials for use in compositions according to the invention include Silkflo 362NF, Silkflo 364NF, and Silkflo 366NF, available from Albermarle Corporation.

When used in compositions according to the invention, the poly olefin hydrocarbons described help to confer to the composition surprisingly good sensory properties, including a surprising lack of greasiness after application, and where the composition is an antiperspirant composition, provide an enhanced degree of masking of any whiteness that may be left by the antiperspirant salt in the composition.

An advantage of using the poly olefin hydrocarbons is that they have been found not to interfere with the efficacy of any antiperspirant active salt in the composition to any major degree.

The non-volatile masking oil which can function as an emollient can also be a non-volatile silicone. The non-volatile silicone may be a polyalkyl siloxane, a polyalkaryl siloxane or a polyether siloxane copolymer. Preferred polyalkysiloxanes have viscosities ranging from 10 to 100,000 mm²s⁻¹ (cSt) at 25°C. Such siloxanes are available from the Dow Corning Corporation as the Dow Corning 200 series.

Suitable polyalkaryl siloxanes are the polymethylphenylsiloxanes having viscosities of 15 to 65mm²s⁻¹ (cSt) at 25°C. These siloxanes are available as Dow Corning 556 fluid. A suitable polyether siloxane is dimethyl polyoxyalkylene ether copolymer having a approximate viscosity of 1200 to 1500 mm²s⁻¹ (cSt) at 25°C e.g. a polysiloxane ethylene glycol ether copolymer.

Other emollients such as non-volatile silicones, hydrocarbons or mineral oils can also be included in the underarm compositions of the invention.

Inert particulates may also be included in cosmetic creams of the present invention. Illustrative of such materials are the polyolefins (such as polyethylene and polypropylene) and nylon. Most preferred are the spherical or non-spherical polyethylene powders. Amounts of these materials may range from 0.1 to 20%, preferably from 1 to 10% by weight.

Waxes may also be incorporated in compositions of the present invention. Animal origin waxes include beeswax, spermaceti, lanolin and shellac wax. Vegetable origin waxes include carnauba, candelilla, bayberry and sugarcane wax. Further examples include high and low melting point waxes, gums, resins, polymers, starches and elastomers. High melting point waxes include insect and animal waxes such as beeswax and spermaceti; vegetable waxes such as carnauba, candelilla, Ouricury, Japan wax, Douglas-fir bark wax, rice-bran wax, castor wax and bayberry wax; mineral waxes such as montan wax, peat wax, ozokerite and ceresin; petroleum waxes such as paraffin wax; synthetic waxes such as Fischer-Tropsch waxes, polyethylene waxes, chemically modified hydrocarbon waxes and substituted amide waxes. Examples of low melting point waxes include fatty acids, fatty alcohols, fatty acid esters and fatty acid amides having carbon chains of 3 to 30 carbon atoms. Particularly preferred low melting point waxes include stearyl alcohol, cetyl alcohol, myristyl alcohol and palmitic acid.

Amounts of the wax may range from 0.5 to 30% by weight.

Most preferably fluid compositions of the present invention which are creams will have a cone penetration value ranging from 2 from 36 mm, preferably from 10 to 25 mm, optimally from 12 to 20 mm as measured in the Standard Test Method for Cone Preparation of Petrolatum (ASTM D 937).

Where the composition is in the form of an anhydrous suspension, other ingredients common in antiperspirant compositions may be included in the composition according to the invention. For example, particularly for the roll-on formulation, an emollient fluid may be present at from 0 to 25% by weight, preferably 1 to 23%, and more preferably 5 to 20%. Suitable emollients are dipropylene glycol methyl ether, a non-volatile silicone fluid material, hydrocarbons such as polydecene or mineral oils.

The compositions according to the invention may also be in the form of an aerosol concentrate which can be diluted with a propellant such as isobutane, butane, propane or pentane used singly or admixed. When used in an aerosol, the amount of the components generally are adjusted by dilution with the propellant to fall within the following ranges (percentages are by weight):
antiperspirant active up to 20%, preferably 1 to 10%, more preferably 5 to 10%, and especially 8 to 10%;
volatile silicone 0.5 to 40%, preferably 10 to 20%;

Where the antiperspirant is in the form of an aerosol, zirconium compounds are not generally appropriate actives.

The following examples will more fully illustrate embodiments of this invention, all percentages being by weight unless otherwise noted.

### EXAMPLE 1

The formulation listed in Table 1 was prepared to evaluate its efficacy and tendency for leakage.

**TABLE 1**

| **FORMULATION** | |
|---|---|
| **COMPONENT** | **WEIGHT %** |
| Cyclomethicone | 39.2 |
| Gransil SR-CYC | 20.0 |
| Polydecene 364 | 14.8 |
| AZAG Salt | 26.0 |

Two test methods were utilized to evaluate leakage. The first is a modified paper chromatography method and the second is a finished product/package test.

In the paper chromatography test, a series of 10 cc beakers were filled with test product. These were then placed into a chromatography development chamber. Instead of standard chromatography paper, fragrance blotter sticks were employed because they were commercially available pre cut with two measurement lines. The blotters were immersed in product up to the first line. They were allowed to stay in the development chamber for at least one hour. Leakage was then measured as the distance from the first line to the leading edge of the liquid front.

For the product/package tests, a standard propel/repel antiperspirant stick dispenser package was filled with product. This product was then extruded above the surface of the package top. Care was taken not to relieve any pressure that built-up inside the dispenser. The top was replaced and the dispenser was allowed to stand for a period of time. Leakage was then observed.

### EXAMPLES 2-8

Illustrative of compositions falling within the present invention are the formulations recorded in Table II.

**TABLE II**

| **COMPONENT** | **EXAMPLE (WEIGHT %)** | | | | | | |
|---|---|---|---|---|---|---|---|
| | 2 | 3 | 4 | 5 | 6 | 7 | 8 |
| Cyclomethicone | 29.7 | 29.7 | 29.7 | 29.7 | 68.0 | 50.0 | 50.0 |
| Gransil SR-CYC | 13.5 | 13.5 | 13.5 | 13.5 | 6.0 | 2.0 | 1.0 |
| Polydecene 364 | 10.8 | 10.8 | 10.8 | 10.8 | -- | 10.8 | 10.8 |
| AZAG | 26.0 | 26.0 | 26.0 | -- | 26.0 | 26.0 | 26.0 |
| Trichlosan | -- | -- | -- | 2.0 | -- | -- | -- |
| Corn Starch | 20.0 | 00 | 18.5 | 20.0 | -- | 11.2 | -- |
| Talc | -- | 20.0 | -- | 20.0 | -- | -- | 10.2 |
| Fumed Silica | -- | -- | 1.5 | 4.0 | -- | -- | 1.0 |

### EXAMPLE 9

### Comparative Example:

A silicone wax based antiperspirant cream having the following formulation was prepared:

| Ingredient | Function | %wt |
|---|---|---|
| Volatile Silicone | Carrier | 43.0 |
| Ethylflo 364 (polydecene) | Deposit Masker | 14.0 |
| Talc | Aid dry skin feel | 6.0 |
| AZAG | Active | 24.0 |
| Perfume | | 1.0 |
| Bentone 38 | Suspending Agent | 1.0 |
| Silicone wax ex GE | | 6.0 |
| Castor Wax MP 80 | Structurant | 5.0 |
| | | |

### Example 10

An anhydrous antiperspirant cream of the invention was prepared having the following formulation:

| Ingredient | Function | % wt |
|---|---|---|
| AZAG (Powder) | Antiperspirant | 24.0 |
| Castorwax MP80 | Structurant | 5.0 |
| Silicone gel | Gellant & | 49.0 |
| JK 301 (C30+) ex GE | Deposit Masker | |
| Bentone | Structurant | 1.0 |
| Non-Volatile Hydrocarbon (Albemarle Ethylflo 364NF) | Deposit Masker | 14.0 |
| Talc (Suprafino) | Aid Dry Skin-Feel | 6.0 |
| Fragrance | | 1.0 |
| Overall % Elastomer | | 2.45% |

The cream was made by heating the wax and liquids to 85°C followed by shearing. The silicone gel was added during shearing followed by the powders and fragrance. Shearing was continued until homogeneous. The resulting product was a thick cream.

### Example 11

An anhydrous antiperspirant cream having the following composition was prepared as described in Example 10:

| Ingredient | %wt |
|---|---|
| AZAG (powder) | 24.0 |
| Castorwax MP80 | 5.0 |
| GE JK301 gel | 33.0 |
| Bentone | 1.0 |
| Volatile silicone DC344 | 16.0 |
| Ethylflo 364NF | 14.0 |
| Talc | 6.0 |
| Fragrance | 1.0 |
| Overall % elastomer | 1.68% |

The resulting product was a cream having a novel dry feel.

### Example 12

| Ingredient | %wt |
|---|---|
| AZAG (powder) | 24.0 |
| GE Silicone gel JK301 | 20.0 |
| Bentone | 1.0 |
| Volatile Silicone DC344 | 34.0 |
| Silkflo 364 NF | 14.0 |
| Talc | 6.0 |
| Fragrance | 1.0 |
| Overall % elastomer | 1.0% |

The resulting product had a thinner consistency with a dry feel.

### Rheology

The rheology of the formulations of example 10, 11, and 12 was examined to demonstrate the stability characteristics of the composition when processed at various temperatures as compared with the silicone wax based composition of the comparative example.

A Carri-Med Controlled Stress Rheometer CSL100 instrument was used. A vane/cup measuring system was employed with a mesh interior fitted to the cup to ensure prevention of wall slip. Experiments were carried out at 25°C unless otherwise stated. Equilibrium flow experiments were conducted on fresh samples to obtain the exact flow properties of the sample. The sample was allowed to equilibrate at a fixed temperature (25°C) for one hour before measuring to ensure no temperature gradient.

The data produced resulted in an equilibrium viscosity figure (Pa.s) which occurs under low shear stress and the point at which the sample catastrophically fails. The lower the equilibrium viscosity figure the more unstable the sample.

### Results

Samples prepared with the silicone elastomer had an improved yield stress compared with the wax based comparative example.

### Stability

The stability of the formulations of examples 11 and 12 over time and at high temperature was compared with the stability of the formulation of the comparative example.

All compositions were packaged in identical dispensing packages.

All formulations were subjected to shear for two minutes at room temperature. The composition of the comparative example became thinner and showed signs of volatile silicone leakage.
However, the compositions of examples 10 and 11 showed no signs of volatile silicone leakage.

The comparative example and examples 10 and 11 were also stored at 40°C for six weeks. The compositions of the comparative example showed silicone leakage. No such leakage occurred with the compositions of the invention.

### Example 13

### Comparative Example:

An antiperspirant roll-on having the following formulation was prepared:

| Ingredient | Function | % wt |
|---|---|---|
| Volatile Silicone (DC344) | Carrier | 59.0 |
| AZAG | Active | 25.0 |
| Bentone | Suspending Agent | 3.0 |
| Polyethylene | Skin Feel Agent | 7.0 |
| Isopropyl Myristate | Emollient | 3.0 |
| Propylene Carbonate | Suspending Agent | 1.0 |
| Aerosil 200 | Suspending Thickening Agent | 0.3 |
| DC200/300 | Emollient | 0.7 |
| Perfume | | 1.0 |

The following examples of roll-on antiperspirant compositions in accordance with the invention were also prepared:

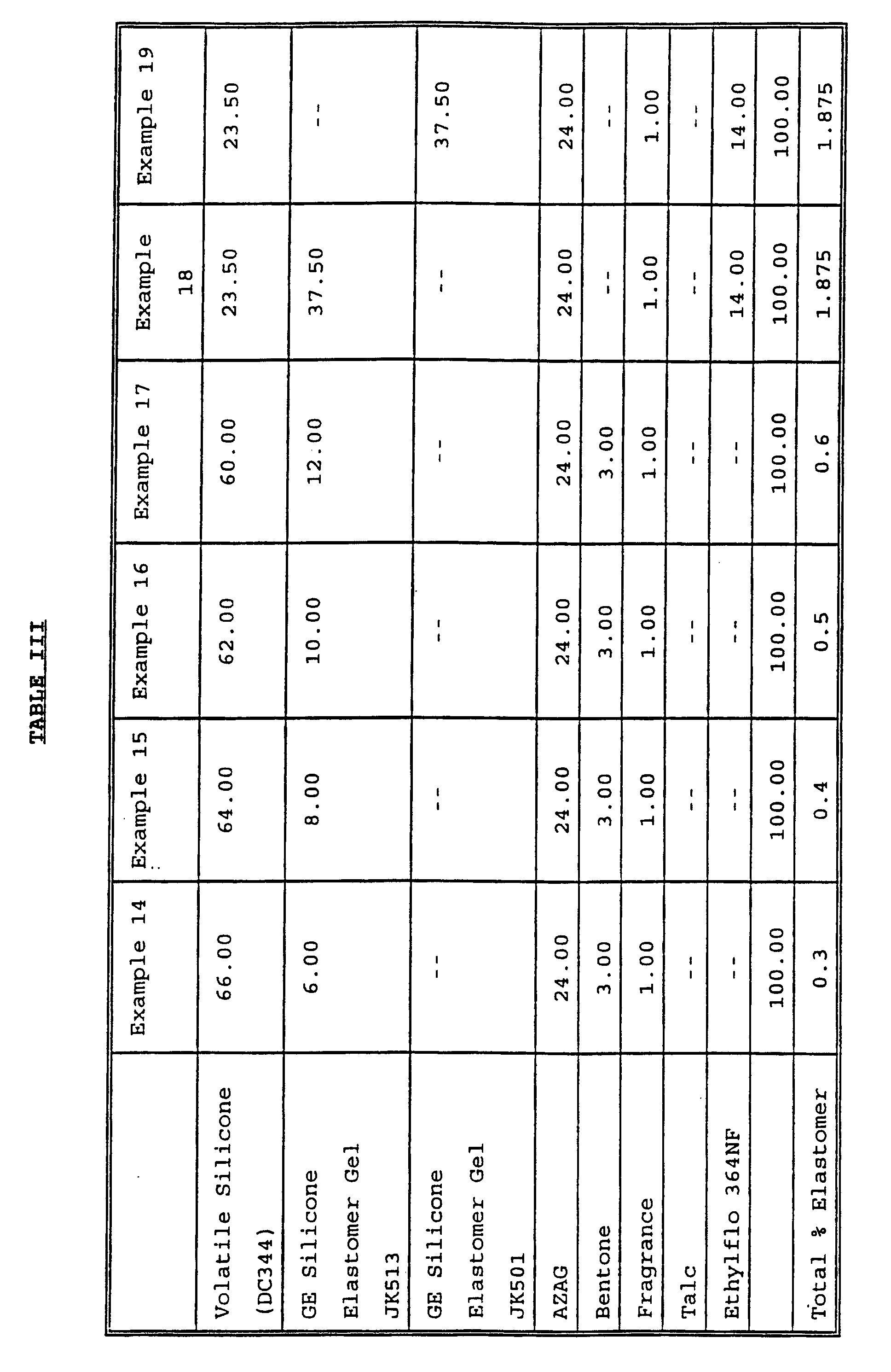

If the dispenser of choice is an aerosol, the present compositions are combined with an aerosol propellant.

The amount of the propellant gas is governed by normal factors as well known in the aerosol art. The composition described previously herein serves as the concentrate and comprises from 7% to 60%, preferably 20% to 40% of the present total aerosol composition while the propellant comprises from 40% to 93%, preferably from 60% to 80% by weight of the composition.

When utilized in aerosol composition the bentone suspending agent is not required. An aerosol composition containing 20% gel was found to be particularly effective. The resulting aerosol has a desirable dry feel. Typically, an aerosol composition having up to 0.25% elastomer is effective while a level of 0.125% elastomer was found to be particularly stable showing little or no separation after three days.

### STABILITY/SEPARATION STUDIES:

The formulations of the comparative example and examples 14 to 19 were examined for separation characteristics. The results are present in Table IV.

The compositions were prepared and examined for separation after 1,2,3,5 and 6 days. As shown the compositions of the invention exhibited significantly less separation than the comparative example thickened with aerosil alone.

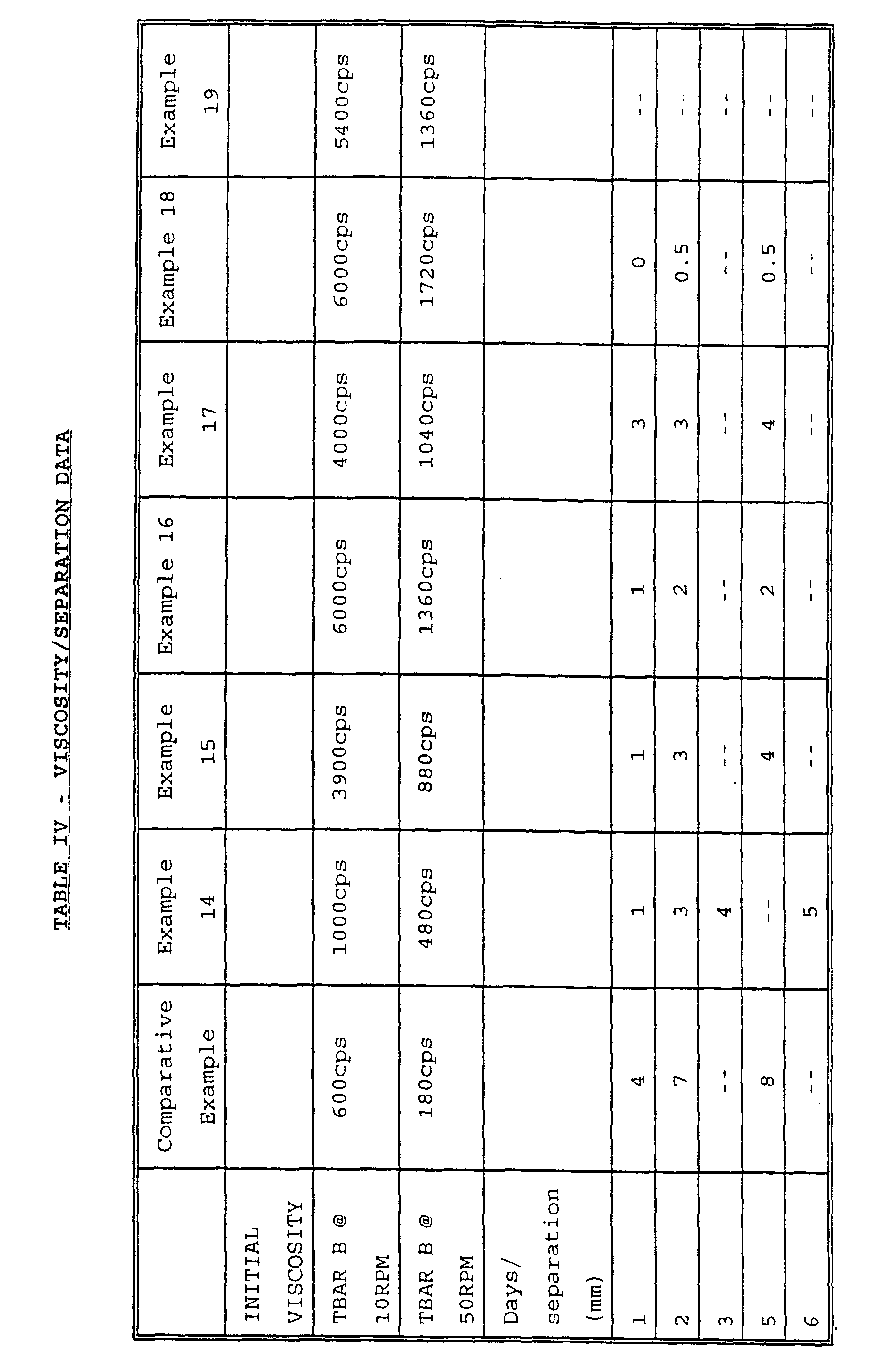

## Claims

1. A fluid underarm treatment composition comprising:
(I) an underarm active present in an effective amount to inhibit odour or to reduce perspiration;
(ii) from 0.1 to 30% by weight of a crosslinked non-emulsifying siloxane elastomer;
(iii) from 10 to 80% by weight of a volatile siloxane.

2. A composition according to claim 1 wherein the crosslinked non-emulsifying siloxane elastomer is formed from a divinyl monomer reacting with Si-H linkages of a siloxane backbone.

3. A composition according to claim 1 or claim 2 which is a cream having a cone penetration value ranging from 10 to 36 mm.

4. A composition according to any one of the preceding claims further comprising from 1 to 40% by weight of a non-volatile C₁₂-C₄₀ hydrocarbon.

5. A composition according to claim 4 wherein the hydrocarbon is polydecene.

6. A composition according to any one of the preceding claims wherein the underarm active is present from 0.1 to 70% by weight.

7. A composition according to any one of the preceding claims wherein the underarm active is an astringent salt of a metal selected from the group consisting of aluminum, zirconium, zinc and mixtures thereof.

8. A composition according to any one of the preceding claims further comprising from 0.5 to 30% by weight of a wax.

9. A composition according to any one of the preceding claims wherein the cross linked siloxane elastomer is formed from the hydrosilysation of vinyl silicone fluids by hydrosiloxane or MQ hydride fluids.

10. A composition according to claim 9 wherein the elastomer is a dimethicone/vinyl dimethicone cross polymer.

11. A composition according to any one of the preceding claims which is a cream having a cone penetration value ranging from 20-36mm.

12. A composition according to any one of the preceding claims further comprising 0.5-30% by weight of a wax.

13. An aerosol composition comprising 7-60% by weight of a composition according to any of the preceding claims and 40-93% propellant.

14. A cream underarm treatment composition according to claim 1 comprising:
(I) an deodorant/antiperspirant active present in an effective amount to inhibit odour or to reduce perspiration;
(ii) from 0.1 to 50% by weight of a cross-linked or partially cross-linked non-emulsifying siloxane elastomer;
(iii)from 10 to 80% by weight of a volatile siloxane.

15. An anhydrous fluid suspension antiperspirant composition according to claim 1 suitable for topical application to human skin, comprising:
I. an effective amount of an antiperspirant astringent;
ii. a volatile silicone; and
iii. a cross-linked or partially cross-linked non-emulsifying siloxane elastomer.

16. A fluid suspension antiperspirant composition according to claim 1 suitable for topical application to the human skin, comprising:
I. 5-25% by weight of the total composition of an antiperspirant astringent;
ii. 10-80% by weight of the total composition of a linear or cyclic volatile silicone;
iii. 0.1 to 20% by weight of a dimethicone/vinyldimethicone cross polymer elastomer thickening/suspending agent, and
iv. a masking oil.

## Patentansprüche

1. Fluides Achselbehandlungsmittel, umfassend:
(I) einen Achselwirkstoff, der in einer wirksamen Menge vorliegt, um Geruch zu hemmen oder Transpirieren zu vermindern;
(ii) 0,1 bis 30 Gew.-% eines vernetzten nicht emulgierenden Siloxanelastomers;
(iii) 10 bis 80 Gew.-% eines flüchtigen Siloxans.

2. Mittel nach Anspruch 1, wobei das vernetzte nicht emulgierende Siloxanelastomer aus einem mit den Si-H-Bindungen eines Siloxangerüsts reagierenden Divinylmonomer hergestellt ist.

3. Mittel nach Anspruch 1 oder Anspruch 2, das eine Creme mit einem Kegelpenetrationswert im Bereich von 10 bis 36 mm darstellt.

4. Mittel nach einem der vorangehenden Ansprüche, weiterhin umfassend 1 bis 40 Gew.-% eines nicht flüchtigen C₁₂-C₄₀-Kohlenwasserstoffs.

5. Mittel nach Anspruch 4, wobei der Kohlenwasserstoff Polydecen ist.

6. Mittel nach einem der vorangehenden Ansprüche, wobei der Achselwirkstoff von 0,1 bis 70 Gew.-% vorliegt.

7. Mittel nach einem der vorangehenden Ansprüche, wobei der Achselwirkstoff ein adstringierendes Salz eines Metalls, ausgewählt aus der Gruppe, bestehend aus Aluminium, Zirkonium, Zink und Gemischen davon, ist.

8. Mittel nach einem der vorangehenden Ansprüche, das weiterhin 0,5 bis 30 Gew.-% eines Wachses umfasst.

9. Mittel nach einem der vorangehenden Ansprüche, wobei das vernetzte Siloxanelastomer aus der Hydrosilylierung von Vinylsilikonfluids durch Hydrosiloxan oder MQ-Hydridfluids gebildet wird.

10. Mittel nach Anspruch 9, wobei das Elastomer ein vernetztes Dimethicon/Vinyldimethicon-Polymer ist.

11. Mittel nach einem der vorangehenden Ansprüche, das eine Creme mit einem Kegelpenetrationswert im Bereich von 20-36 mm ist.

12. Mittel nach einem der vorangehenden Ansprüche, das weiterhin 0,5 bis 30 Gew.-% eines Wachses umfasst.

13. Aerosolmittel, umfassend 7 bis 60 Gew.-% eines Mittels nach einem der vorangehenden Ansprüche und 40 bis 93 % Treibmittel.

14. Creme-förmiges Achselbehandlungsmittel nach Anspruch 1, umfassend:
(I) einen Deodorant/Antitranspirant-Wirkstoff, der in einer wirksamen Menge vorliegt, um Geruch zu hemmen oder Transpiration zu vermindern;
(ii) 0,1 bis 50 Gew.-% eines vernetzten oder teilweise vernetzten nicht emulgierenden Siloxanelastomers;
(iii) 10 bis 80 Gew.-% eines flüchtigen Siloxans.

15. Wasserfreies fluides Suspensionsantitranspirantmittel nach Anspruch 1, geeignet zur örtlichen Auftragung auf die Haut des Menschen, umfassend:
I. eine wirksame Menge eines Antitranspirant-Adstringents,
ii. ein flüchtiges Silikon und
iii. ein vernetztes oder teilweise vernetztes nicht emulgierendes Siloxanelastomer.

16. Fluides Suspensionsantitranspirantmittel nach Anspruch 1, geeignet zur örtlichen Auftragung auf die Haut des Menschen, umfassend:
I. 5-25 Gew.-% des gesamten Mittels eines Antitranspirant-Adstringents,
ii. 10-80 Gew.-% des gesamten Mittels eines linearen oder cyclischen flüchtigen Silikons,
iii. 0,1-20 Gew.-% eines Dimethicon/Vinyldimethicon-Vernetzungs-Polymer-Elastomer-Verdickungs/Suspendierungsstoffs, und
iv. ein maskierendes Öl.

## Revendications

1. Composition pour le traitement des aisselles sous forme fluide comprenant:
(i) un principe actif pour aisselle présent en une quantité efficace pour inhiber l'odeur ou pour réduire la transpiration;
(ii) de 0,1 à 30% en poids d'un élastomère siloxane non émulsifiant réticulé;
(iii) de 10 à 80% en poids d'un siloxane volatile.

2. Composition selon la revendication 1, dans laquelle l'élastomère siloxane non émulsifiant réticulé est formé à partir d'un monomère divinylique réagissant avec les liaisons Si-H d'un squelette siloxane.

3. Composition selon la revendication 1 ou 2, qui est une crème ayant une valeur de pénétration de cône comprise entre 10 et 36 mm.

4. Composition selon l'une quelconque des revendications précédentes, comprenant de plus de 1 à 40% en poids d'un hydrocarbure en C₁₂ à C₄₀ non volatile.

5. Composition selon la revendication 4, dans laquelle l'hydrocarbure est le polydécène.

6. Composition selon l'une quelconque des revendications précédentes, dans laquelle le principe actif pour aisselles est présent de 0,1 à 70% en poids.

7. Composition selon l'une quelconque des revendications précédentes, dans laquelle le principe actif pour aisselles est un sel astringent d'un métal choisi dans le groupe formé par l'aluminium, le zirconium, le zinc et leurs mélanges.

8. Composition selon l'une quelconque des revendications précédentes comprenant de plus de 0,5 à 30% en poids d'une cire.

9. Composition selon l'une quelconque des revendications précédentes, dans laquelle l'élastomère siloxane réticulé est formé à partir de l'hydrolyse de fluides de vinylsilicone par l'hydrosiloxane ou les fluides d'hydrure MQ.

10. Composition selon la revendication 9, dans laquelle l'élastomère est un polymère réticulé diméthicone/vinyldiméthicone.

11. Composition selon l'une quelconque des revendications précédentes, qui est une crème ayant une valeur de pénétration de cône comprise entre 20 et 36 mm.

12. Composition selon l'une quelconque des revendications précédentes, comprenant de plus 0,5 à 30% en poids d'une cire.

13. Composition aérosol comprenant de 7 à 60% en poids d'une composition selon l'une quelconque des revendications précédentes et 40 à 93% de propulseur.

14. Composition pour le traitement des aisselles sous forme de crème selon la revendication 1 comprenant:
(i) un principe actif déodorant/anti-transpirant présent en une quantité efficace pour inhiber l'odeur ou pour réduire la transpiration;
(ii) de 0,1 à 50% en poids d'un élastomère siloxane non émulsifiant réticulé ou partiellement réticulé;
(iii) de 10 à 80% en poids d'un siloxane volatile.

15. Composition anti-transpirante en suspension sous forme de fluide anhydre selon la revendication 1, appropriée pour une application topique à la peau humaine, comprenant:
(i) une quantité efficace d'un astringent anti-transpirant;
(ii) une silicone volatile; et
(iii) un élastomère siloxane non émulsifiant réticulé ou partiellement réticulé.

16. Composition anti-transpirante en suspension sous forme de fluide selon la revendication 1, appropriée pour une application topique à la peau humaine, comprenant:
(i) 5% à 25% en poids de la composition totale d'un astringent anti-transpirant;
(ii) 10% à 80% en poids de la composition totale d'une silicone volatile linéaire ou cyclique;
(iii) 0,1% à 20% en poids d'un agent épaississant/de suspension élastomère polymère réticulé diméthicone /vinyldiméthicone; et
(iv) une huile masquante.
